# EUROPÄISCHE PATENTANMELDUNG

(11) **EP 1 801 719 A1**
(43) Veröffentlichungstag der Anmeldung: **27.06.2007**
(21) Anmeldenummer: 05090356.6
(22) Anmeldetag: 21.12.2005
(51) Int. Cl.: G06F 19/00

(54) **Computerimplementiertes Verfahren zur Distribution von pharmazeutischen und medizinischen Produkten**

(71) Anmelder: Körber AG, 20097 Hamburg (DE)
(72) Erfinder: Strub, Nikolai, 24568 Kaltenkirchen (DE); von Bismarck, Gottfried Dr., 20149 Hamburg (DE); Breu, Gerhard, 8594 Güttingen (CH)
(74) Vertreter: Wenzel & Kalkoff

(57) **Zusammenfassung**

Die Erfindung betrifft ein computerimplementiertes Verfahren zur Distribution von pharmazeutischen und medizinischen Produkten (50) und Nahrungsergänzungsmitteln, das sich dadurch auszeichnet, dass mehrere pharmazeutische oder medizinische Produkte (50) oder Nahrungsergänzungsmittel in vorkonfektionierten Blisterverpackungen bereitgestellt werden, wobei jede Blisterverpackung ausschließlich Produkte (50) eines speziellen Wirkstoffes oder einer speziellen Wirkstoffkombination in einer speziellen Dosierung trägt, diese vorkonfektionierten Blisterverpackungen auftragsgesteuert zu einer patientenindividuellen Verpackung (20) zusammengestellt werden, und die patientenindividuelle Verpackung (20) an den Auftraggeber (11) geliefert wird.

## Beschreibung

Die Erfindung betrifft ein computerimplementiertes Verfahren zur Distribution von pharmazeutischen und/oder medizinischen Produkten und/oder Nahrungsergänzungsmitteln.

Bisher werden Verpackungen mit pharmazeutischen und/oder medizinischen Produkten überwiegend direkt vom Hersteller in Standard-Verpackungen abgefüllt und an entsprechende Importeure und/oder Pharmahandelsunternehmen und/oder Logistikeinheiten oder dergleichen geliefert. Die Apotheken beziehen die Verpackungen dann auf Kundenwunsch z.B. von den Logistikeinheiten, wobei die Verpackungen in ihrer Produktkombination und Menge nicht auf die spezifischen Patienten abgestimmt sind. Anders ausgedrückt werden beispielsweise N1-, N2- oder N3-Verpackungen einer bestimmten Tablette produziert und geliefert. Das bedeutet, dass bei einem Patienten, der aufgrund seiner Beschwerden weniger Tabletten benötigt, als in einer Verpackung vorhanden sind, Tabletten übrig bleiben, die nach Ablauf des Verfalldatums in der Regel vernichtet werden. In anderen Fällen benötigen Patienten z.B. zwei unterschiedliche Medikamente, die dann in zwei Verpackungen geliefert werden.

Es ist daher zunehmend erforderlich und gewünscht, patientenindividuelle Verpackungen anzubieten, um einerseits eine abgestimmte Therapie durchführen zu können und anderseits aus Kostengründen die Ausgabe von Verpackungen mit zu großer Anzahl von Produkten zu verhindern. Aus diesem Grunde werden bereits vereinzelt individuelle Verpackungen produziert, in denen Produkte unterschiedlicher Hersteller angeordnet sind, wobei die Verteilung der pharmazeutischen und/oder medizinischen Produkte problematisch ist.

Die Anlieferung und Verteilung von Rohware ist aus hygienischen und rechtlichen Gründen schwierig. Das Entpacken insbesondere von pharmazeutischen und/oder medizinischen Produkten bei der Umfüllung in die individuellen Verpackungen erfordert ebenfalls Reinraumbedingungen. Ein weiteres Problem besteht darin, dass jeder Dritte, der Produkte entpackt und wieder verpackt, als Hersteller solcher Produkte gilt, für den entsprechende gesetzliche Bestimmungen gelten. Damit ist die bekannte Vorgehensweise an aufwendige Randbedingungen geknüpft, deren Erfüllung langwierig und teuer ist. Auch ist die derzeitige Bereitstellung der Produkte ungeeignet, eine schnelle und sichere Zusammenstellung verschiedener Produkte zu einer individuellen Verpackung zu gewährleisten.

Es ist daher Aufgabe der vorliegenden Erfindung, ein vereinfachtes und kostengünstiges Verfahren zur Distribution von pharmazeutischen und/oder medizinischen Produkten vorzuschlagen.

Diese Aufgabe wird durch ein eingangs genanntes Verfahren dadurch gelöst, dass mehrere pharmazeutische und/oder medizinische Produkte und/oder Nahrungsergänzungsmittel in vorkonfektionierten Blisterverpackungen bereitgestellt werden, wobei jede Blisterverpackung ausschließlich Produkte eines speziellen Wirkstoffes oder einer speziellen Wirkstoffkombination in einer speziellen Dosierung trägt, diese vorkonfektionierten Blisterverpackungen auftragsgesteuert zu einer patientenindividuellen Verpackung zusammengestellt werden, und die patientenindividuelle Verpackung an den Auftraggeber geliefert wird. Mit diesem Verfahren ist eine besonders schnelle und zuverlässige Versorgung der Auftraggeber mit den individuellen Verpackungen gewährleistet. Als Auftraggeber können für rezeptfreie und nicht apothekenpflichtige Produkte insbesondere die Patienten selbst, ansonsten insbesondere Ärzte, Apotheken, Kliniken oder andere Institutionen auftreten.

Vorzugsweise liefern die Hersteller der pharmazeutischen und/oder medizinischen Produkte diese in vorkonfektionierten und maschinengängigen Blistern als Gurt, Magazin oder in jedem anderen gängigen Blistertyp in aufgerollter, gefalteter oder anderweitig konfektionierter Anordnung an ein Verteilungszentrum, das die Verpackungen herstellt und an die Auftraggeber liefert. Dies stellt zum einen eine kostengünstige und fehlerarme Konfektionierung sicher. Zum anderen lässt sich durch die Bereitstellung der Produkte in maschinengängigen Blistereinheiten besonders wirtschaftlich und zeitnah eine patientenindividuelle Verpackung herstellen, in der die Produkte für einen definierten Zeitraum patientengerecht und patientenfreundlich angeordnet sind. Selbstverständlich sind auch andere maschinengängige Träger, Verpackungstypen etc. einsetzbar.

Vorteilhafterweise ist das oder jedes die Verpackungen herstellende Verteilungszentrum mit Arztpraxen und/oder Kliniken und/oder Apotheken und/oder anderen berechtigten Institutionen als Auftraggeber vernetzt. Dadurch können die Aufträge patientenindividuell erfasst und die entsprechenden Verpackungen kurzfristig hergestellt werden. Idealerweise vergehen von einer Bestellung einer individuellen Verpackung bis zur Auslieferung an den Auftraggeber nur wenige Stunden.

Eine bevorzugte Weiterbildung des Verfahrens ist dadurch gekennzeichnet, dass die Arztpraxen, Kliniken, Apotheken, andere berechtigte Personen und Institutionen als Auftraggeber sowie die Hersteller der pharmazeutischen und/oder medizinischen Produkte, übergeordnete und regionale Verteilungszentren und Logistikeinheiten untereinander vernetzt sind. Dies ermöglicht eine bedarfsgesteuerte und/oder auftragsgesteuerte Lieferung an das oder jedes die Verpackungen herstellende Verteilungszentrum.

Weitere bevorzugte oder vorteilhafte Verfahrensschritte und -abläufe ergeben sich aus den Unteransprüchen und der Beschreibung. Besonders bevorzugte Verfahrensabläufe werden anhand der beigefügten Zeichnung näher erläutert. In der Zeichnung zeigt:
- Fig. 1: ein schematisches Blockdiagramm für eine Distributionsstruktur, in der mehrere Hersteller ein Verteilungszentrum direkt mit vorkonfektionierten Produkten versorgen, und
- Fig. 2: ein schematisches Blockdiagramm für eine mehrstufige Distributionsstruktur.

Die beschriebenen logistischen Verfahren dienen zur patientenspezifischen Distribution von pharmazeutischen und/oder medizinischen Produkten 50. Das erfindungsgemäße Verfahren wird anhand der Distribution von Tabletten beschrieben, gilt jedoch auch für Dragees, Ampullen, Spritzen oder andere in Blisterverpackungen passende Produkte.

Bei dem allgemeinen Verfahrensprinzip werden mehrere Tabletten 50 in vorkonfektionierten Blisterverpackungen bereitgestellt. Jede Blisterverpackung trägt ausschließlich Produkte eines speziellen Wirkstoffes oder einer speziellen Wirkstoffkombination in einer speziellen Dosierung. Das bedeutet, dass auch Produkte, die dieselbe Wirkstoffkombination jedoch eine unterschiedliche Dosierung aufweisen, in unterschiedlichen Blisterverpackungen angeordnet sind. Aus der Vielzahl der bereit stehenden, vorkonfektionierten Blisterverpackungen werden je nach Auftrag patientenspezifische Verpackungen 20 zusammengestellt. Dazu werden die auftragsabhängigen Mengen von den einzelnen Blisterverpackungen abgetrennt und unter Bildung eines Blisterverbundes zu einer individuellen Verpackung 20 zusammengestellt. Die patientenspezifischen Verpackungen 20 werden dann an den Auftraggeber oder den Patienten direkt geliefert. Wenn in diesem Zusammenhang von Blisterverpackungen gesprochen wird, beinhaltet dies auch andere Verpackungstypen.

### Konkret kann das Verfahren z.B. wie folgt ablaufen:

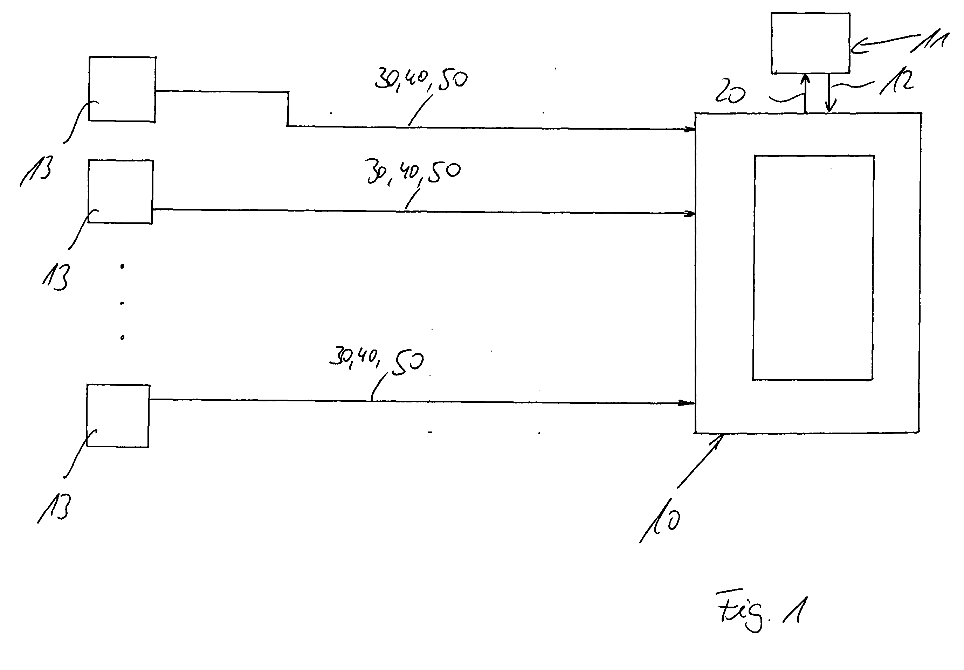

Bei dem Verfahren gemäß Figur 1 stellt ein Verteilungszentrum 10 patientenindividuelle Verpackungen her. Ein Auftraggeber 11 liefert die für die Zusammenstellung der Verpackung notwendigen Informationen 12. Die Informationen 12 können mündlich, schriftlich oder üblicherweise in elektronsicher Form übermittelt werden. Als Auftraggeber 11 können Patienten selbst fungieren, solange es sich um rezeptfreie und nicht apothekenpflichtige Produkte/Tabletten handelt. In der Regel enthalten die Informationen 12 aber Rezeptdaten, die von einem Arzt erstellt und durch diesen oder eine Apotheke direkt an das Verteilungszentrum 10 übermittelt werden.

Die für die Zusammenstellung der gewünschten, nach Inhalt und Menge individuellen Verpackung erforderlichen Tabletten können dem Verteilungszentrum 10 direkt von unterschiedlichen Herstellern 13 geliefert werden. Für den Fall, dass die Tabletten als Rohware 40 an das Verteilungszentrum 10 geliefert werden, füllt das Verteilungszentrum 10 die Tabletten selbst in maschinengängige Blisterverpackungen, wie z.B. Blistergurte 30, so dass die Tabletten in den vorkonfektionierten Blistergurten 30 zur weiteren Verarbeitung vorliegen. Dabei ist jedem Blistergurt 30 eine Vielzahl von Produkten 50 eines speziellen Wirkstoffes oder einer speziellen Wirkstoffkombination mit einer speziellen Dosierung zugeordnet. Auf dem Blistergurt 30 selbst ist jedem Nest des Blistergurtes 30 nur eine Tablette des Produktes 50 zugeordnet. Aus diesen vorkonfektionierten Blistergurten 30 wird dann die individuelle Verpackung zusammengestellt. Für den üblichen Fall, dass die Tabletten direkt beim eigentlichen Produzenten der Tabletten in vorzugsweise maschinengängige Blisterverpackungen, wie z.B. die Blistergurte 30 gefüllt werden, liefern die Hersteller 13 die Blistergurte 30 an das Verteilungszentrum 10.

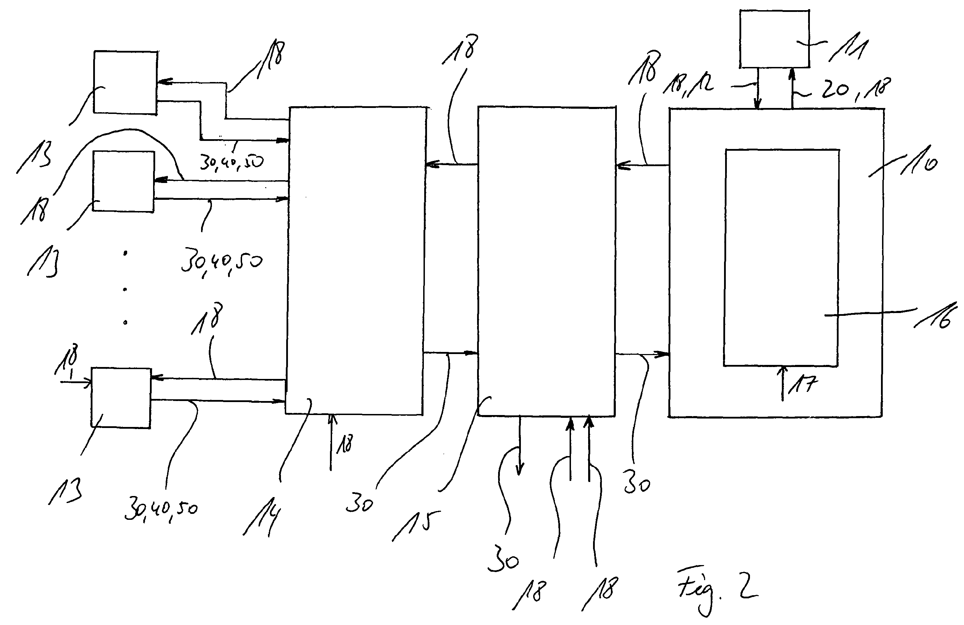

Je nach Einzugs- bzw. Versorgungsgebiet ist das Verteilungszentrum 10 regional ausgerichtet. Das bedeutet, dass das Verteilungszentrum 10 die Zusammenstellung und Auslieferung der individuellen Verpackungen 20 in einem örtlich definierten Umkreis gewährleistet. In der Regel existieren mehrere regionale Verteilungszentren 10, die direkt von den Herstellern 13 beliefert werden können. Es kann jedoch auch ein überregionales, nationales oder internationales Verteilungszentrum 14 (siehe z.B. Figur 2) vorgesehen sein. Das übergeordnete Verteilungszentrum 14 erhält die Tabletten von einem oder mehreren Herstellern 13 wiederum als Rohware 40 und/oder in Blistergurten 30 verpackt. Die Blistergurte 30 und die vom Verteilungszentrum 14 mit der Rohware 40 befüllten Blistergurte 30 werden dann entweder direkt an einen oder mehrere regionale Verteilungszentren 10 und/oder (wie in Figur 2) an eine Logistikeinheit 15 geliefert, wobei auch die Logistikeinheit 15 in mehrere kleinere Einheiten unterteilt sein kann.

Unabhängig von der Belieferung des die Verpackungen 20 produzierenden Verteilungszentrums 10 hat Letzteres die gängigsten und am häufigsten verschriebenen Produkte 50 sowie die verbreitetsten Kombinationspräparate bevorratet. Das bedeutet, dass aufgrund eingehender Informationen 12 und aus Erfahrungswerten diejenigen Präparate vorgehalten werden, die für die Zusammenstellung individueller Verpackungen 20 benötigt werden. Selbst wenn einzelnen Produkte 50 nicht im Verteilungszentrum 10 vorrätig sein sollten, können diese Produkte 50 kurzfristig vom Hersteller 13 und/oder vom übergeordneten Verteilungszentrum 14 und/oder von der Logistikeinheit 15 bezogen werden.

Wie insbesondere aus der Figur 2 ersichtlich, können die Auftraggeber 11 zum Transfer von Aufträgen mit dem Verteilungszentrum 10 vernetzt sein. Der Transfer der Informationen 12 kann aber auch auf herkömmlichem Wege per e-mail oder auf andere übliche computergestützte Weise erfolgen. Die eingehenden Aufträge können innerhalb des Verteilungszentrums 10 mittels geeigneter Vorrichtungen 16 automatisch und computergesteuert abgearbeitet werden, indem von den bevorrateten Blistergurten 30 die ausgewählten Produkte 50 in vorgegebener Menge abgetrennt und auf einem der Vorrichtung 16 zugeführten Zuschnitt 17, Substrat oder dergleichen abgesetzt werden. Die dadurch entstehenden patientenindividuellen Blisterverbunde bzw. Verpackungen 20 können dann vor der Auslieferung noch beschriftet, codiert oder anderweitig gekennzeichnet werden, insbesondere auch mit elektronischen Hilfsmitteln.

Zusätzlich ist das Verteilungszentrum 10 optional auch mit der Logistikeinheit 15 und/oder dem übergeordneten Verteilungszentrum 14 und/oder den Herstellern 13 verbunden. Diese Verbindung kann durch herkömmliche Kommunikationsmittel hergestellt bzw. durch eine Vernetzung sichergestellt sein. Auch können mehrere Verteilungszentren 10 untereinander vernetzt sein. Durch die Verbindungen können insbesondere Produkte 50 bzw. Blistergurte 30 bedarfsgesteuert und/oder auftragsgesteuert an das oder jedes die Verpackungen 20 herstellende Verteilungszentrum 10 geliefert werden. Auch ist die Bevorratung mit den relevanten Produkten 50 bzw. Blistergurten 30 innerhalb der Stationen 14, 15, 10 einfach sicher zu stellen. Die vorgenannten Verbindungen sind durch die Pfeile 18 angedeutet, wobei sich weitere und zusätzliche Verbindungen selbstverständlich möglich sind.

Die produzierten Verpackungen 20 können durch das Verteilungszentrum 10 selbst oder durch die Logistikeinheit 15 quasi als Kurierdienst an den Auftraggeber 11 oder direkt an den Patienten geliefert werden. Zusätzlich zu den Verpackungen 20 können auch mittels der Verbindung (Pfeil 18) Informationen/Daten vom Verteilungszentrum 10 an den Auftraggeber 11 transferiert werden.

Das zuvor beschriebene Prinzip kann vereinfacht auch wie folgt an einem einfachen Beispiel erläutert werden:
Zur Versorgung von Patienten mit einem patientenindividuell für einen definierten Einnahmezeitraum zusammengestellten Bedarf an einem Medikament A und/oder einem Medikament B wird vorgeschlagen, das Medikament A in einer eine Vielzahl von jeweils separat eingehaust beabstandet zueinander angeordnete Exemplare EA des Medikamentes A tragenden Träger C und/oder das Medikament B in einer eine Vielzahl von jeweils separat eingehaust beabstandet zueinander angeordnete Exemplare EB des Medikamentes B tragenden Träger D bereitzustellen, vom Träger C Abschnitte CA mit einem oder mehreren Exemplaren EA des Medikamentes A und/oder vom Träger D Abschnitte DB mit einem oder mehreren Exemplaren des Medikamentes B abzutrennen und die abgetrennten Abschnitte CA und/oder DB in einer matrixartigen Formation auf einem Substrat oder dergleichen einnahmekonform anzuordnen. Diese patientenindividuelle Verpackung kann dem Auftraggeber zugestellt werden.

## Patentansprüche

1. Computerimplementiertes Verfahren zur Distribution von pharmazeutischen und/oder medizinischen Produkten (50) und/oder Nahrungsergänzungsmitteln, **dadurch gekennzeichnet,dass**
a) mehrere pharmazeutische und/oder medizinische Produkte (50) und/oder Nahrungsergänzungsmittel in vorkonfektionierten Blisterverpackungen bereitgestellt werden, wobei jede Blisterverpackung ausschließlich Produkte (50) eines speziellen Wirkstoffes oder einer speziellen Wirkstoffkombination in einer speziellen Dosierung trägt,
b) diese vorkonfektionierten Blisterverpackungen auftragsgesteuert zu einer patientenindividuellen Verpackung (20) zusammengestellt werden, und
c) die patientenindividuelle Verpackung (20) an den Auftraggeber (11) geliefert wird.

2. Verfahren nach Anspruch 1, **dadurch gekennzeichnet, dass** die Hersteller (13) der pharmazeutischen und/oder medizinischen Produkte (50) und/oder Nahrungsergänzungsmittel diese als Rohware (40) an mindestens ein Verteilungszentrum (10) liefern, das die Produkte (50) selbst in Blisterverpackungen abfüllt und dann zu patientenindividuellen Verpackungen (20) zusammenstellt.

3. Verfahren nach Anspruch 1, **dadurch gekennzeichnet, dass** die Hersteller (13) der pharmazeutischen und/oder medizinischen Produkte (50) und/oder Nahrungsergänzungsmittel diese in vorkonfektionierten Blistergurten (30) an ein Verteilungszentrum (10) liefern, das die Verpackungen (20) herstellt und an die Auftraggeber (11) liefert.

4. Verfahren nach Anspruch 3, **dadurch gekennzeichnet, dass** das die Verpackungen (20) herstellende Verteilungszentrum (10) regional ausgerichtet ist und die vorkonfektionierten Blistergurte (30) wahlweise direkt von den Herstellern (13) und/oder von einem übergeordneten Verteilungszentrum (14) und/oder einer Logistikeinheit (15) bezieht.

5. Verfahren nach einem der Ansprüche 2 bis 4, **dadurch gekennzeichnet, dass** das die individuellen Verpackungen (20) herstellende Verteilungszentrum (10) die gängigsten Produkte (50) bevorratet.

6. Verfahren nach Anspruch 4 oder 5, **dadurch gekennzeichnet, dass** das übergeordnete Verteilungszentrum (14) bzw. die daran angeschlossene Logistikeinheit (15) mehrere regionale Verteilungszentren (10) beliefert.

7. Verfahren nach einem der Ansprüche 2 bis 6, **dadurch gekennzeichnet, dass** das oder jedes die Verpackungen (20) herstellende Verteilungszentrum (10) mit Arztpraxen und/oder Kliniken und/oder Apotheken und/oder anderen berechtigten Institutionen als Auftraggeber (11) vernetzt ist.

8. Verfahren nach Anspruch 7, **dadurch gekennzeichnet, dass** die Arztpraxen, Kliniken, Apotheken, andere berechtigte Personen und Institutionen als Auftraggeber (11) sowie die Hersteller (13) der pharmazeutischen und/oder medizinischen Produkte (50), übergeordnete und regionale Verteilungszentren (10, 14) und Logistikeinheiten (15) untereinander vernetzt sind.

9. Verfahren nach einem der Ansprüche 1 bis 8, **dadurch gekennzeichnet, dass** das Verfahren computerunterstützt und voll automatisch ausgeführt wird.
